Europäisches Patentamt

European Patent Office

Office européen des brevets

(11) Publication number: **0 271 157**
**A1**

## (12) EUROPEAN PATENT APPLICATION

(21) Application number: **87202398.1**

(22) Date of filing: **02.12.87**

(51) Int. Cl.⁴: **A61B 1/12** , A61L 2/18

(30) Priority: **03.12.86 NL 8603085**

(43) Date of publication of application:
**15.06.88 Bulletin 88/24**

(84) Designated Contracting States:
**AT BE CH DE ES FR GB GR IT LI LU NL SE**

(71) Applicant: **WASSENBURG & CO. B.V.**
**Groenestraat 14**
**NL-6669 DS Dodewaard(NL)**

(72) Inventor: **Wassenburg Gerrit**
**Groenestraat 14**
**6669 DS Dodewaard(NL)**

(74) Representative: **van der Beek, George Frans**
**et al**
**Nederlandsch Octrooibureau Johan de**
**Wittlaan 15 P.O. Box 29720**
**NL-2502 LS 's-Gravenhage(NL)**

(54) **Device for cleansing and disinfecting an elongated medical instrument.**

(57) Device for disinfecting and cleaning long medical instruments such as an endoscope.

The device comprises a tubular vessel (10) adapted to receive the flexible part of the instrument and a separate detachable part (4) for taking up the head of the instrument. The vessel is helical or spiral for reduced height.

Means are provided to connect the conduits of the instrument and the vessel to a cleaning and disinfecting system made itself to be readily taken apart and reassembled by using hoses (16, 37-41) and hose pumps (54-58).

fig-1

# Device for cleansing and disinfecting an elongated medical instrument.

The invention relates to a device for cleansing and disinfecting an elongated medical instrument of the type with a long flexible part, such as an endoscope, said device comprising an elongated tubular vessel having a cross section which is greater than that of the instrument to be treated, and having a length suitable for taking the flexible part of the instrument to be treated, said vessel having a top end and a bottom end, of which the top end is suitable for introducing an instrument to be treated, and of which the bottom end can be connected to a treatment liquid discharge, said device also being provided with one or more tanks with treatment media and with a channel system with valves, pumps and hoses for the infeed of said media to the inner channels of the instrument to be treated and/or to the top end of the tubular vessel.

Such a device is known under the name "Endo disinfectors type ED-02 and JW-05", put on the market by Fujinon Medical Holland B.V., Renswoude. With this known device it is possible to cleanse and disinfect medical instruments of the endoscope, bronchioscope etc. types internally and externally. Such an instrument to be cleansed comprises a head having thereon a long flexible part containing the channels and generally also an optical fibre. During use, substances can be fed through the channels to the place in the human body to be treated, or they can be removed, or operations can be carried out, or undesirable objects can be removed. Such an instrument must be absolutely sterile before it is used.

In the known device the tubular elongated vessel is a long pipe of transparent material, the greatest length of which is disposed vertically in a frame or cabinet, and which near the bottom end is bent laterally in a slight curve and ends in a part going vertically downwards. This elongated vessel is therefore very long and, due to the essentially vertical arrangement of this vessel which is needed to permit the discharge of treatment liquids through gravity, the device is very high.

The medical instrument to be cleansed is inserted at the top, with the head of the instrument remaining projecting beyond the top end of the pipe and being hung on the cabinet. This head has connections for hoses. The bottom end of the long S-shaped pipe is connected to two collection tanks by means of two pipes provided with valves, so that discharge of the contents of the pipe can take place as desired into one of the two collection tanks. The pipe is filled by means of the medical instrument to be cleansed, to which the hoses mentioned earlier can be connected, or by direct infeed of the liquid to be treated to the top end of the pipe. The reason why the head of the instrument to be sterilized itself remains outside the range of the media to be used is that a number of the known scopes must not be fully immersed.

In the interior of this known device, i.e. in the cabinet of the device is a channel system with valves and pumps for suction of treatment media through that system out of tanks and feeding thereof to a number of connection points for hoses located at the accessible front side of the cabinet, said hoses then being connectable in the manner mentioned earlier to the top end of the S-shaped pipe or to the head of the medical instrument to be cleansed.

This channel system is not readily accessible, is not detachable and is thus not easy to clean, for example using an autoclave. The long S-shaped vessel cannot be cleansed using an autoclave either. This vessel has at the top side an overflow pipe which is connected to the valve system for control of the discharge of the liquid, provided in the interior of the device.

This known device is itself therefore difficult or impossible to keep or render sterile. The disinfectant liquid does not fill the pipe to the top rim, so there always remains a place where infection of the disinfected part of the instrument can take place through the fact that the instrument comes into contact with this place when being removed. With this known device it is not possible to disinfect the head of an instrument on the outside. Sterilization of the long pipe, of the channel system with valves and pumps and of the cabinet is virtually impossible.

The object of the invention is then to produce a device which overcomes these disadvantages.

This object is achieved according to the invention in the first place in that the vessel is detachably connected at the top end to a container whose shape and/or volume is suitable for the accommodation of the operating head of the instrument to be treated. The detachable container at the top end of the tubular vessel means that the head of the instrument to be treated - such as an endoscope to be treated - can also be completely flushed, or on use of the extension piece yet to be discussed, with placing of the head fully or partially outside the container, that the introduction of the long flexible part is facilitated and on removal the sterilized part does not come into contact with the non-sterile parts of the device.

It is also preferable according to the invention if the vessel between top and bottom end follows a path provided with curves, whose width in the horizontal direction is greater than the vertical distance between top and bottom end. This achieves a low construction height and the device is thus easier to operate, due to the fact that the place for introduction of the endoscope by means of the container can come to rest at a normal operating level.

What is also achieved is that the vessel itself is given such small dimensions that it can after all be cleansed in an autoclave.

The path of the vessel can be helical or spiral. With the helical path the tubular vessel then runs in a helical line with a vertical central axis and with a fairly large screw diameter in a plane perpendicular to the central axis, and with a relatively low height. In the case of a spiral path the vessel runs from the outside of the spiral where the top end is located to a more centrally positioned and preferably lower down part which is connected to the discharge of a treatment medium to be determined by the control system.

According to the invention, the container can be closable so that it is liquid-tight, for example by means of a lid, and provision is made in the wall for sealed throughfeed means for the supply of the treatment media through the wall to the container, in particular to an operating head of an instrument for cleansing located in the sealable container. In this embodiment the endoscope to be treated can thus be completely incorporated with its head in the container and can be completely flushed. The infeed of media to the channels of head and flexible part then takes place by means of hoses which in the inside of the container have connections to the connection points of the head of the instrument to be treated.

It is also possible according to the invention for the container to be provided with an extension piece which can be placed on the top rim of the container and forms a support for the head of an instrument to be treated, and which has a passage for the part of the instrument following the head. Such an extension piece can be in the form of a bowl with an opening in the bottom carrying the head to allow through the instrument, and with supports for the optical fibre of the instrument. Cleansing liquid can then cleanse the part of the head projecting into the container. The hose connections can take place through liquid-tight passages through the wall of the container or through the open top side of the bowl. This bowl can, of course, also have a lid to stop the escape of vapours. The liquid will generally remain below the top edge of the container and will thus not pass into the extension piece. A scope which is not immersible is therefore flushed only up to and including part of the head.

According to the invention, the extension can also be a lid having a support for supporting the head of the instrument to be treated on the outside of the lid, and also having a support for the optical fibre on this instrument, said lid having a feed-through slit for the part of the instrument connecting to the head of the instrument. In this embodiment the head is thus entirely outside the device. The interior can be cleansed by connection of the hoses to the channels. The outside cannot be cleansed by the treatment liquid which is fed to the container, but on removal of the treated instrument it will not come into contact with non-sterile parts of the device, because the sterilized part does not come into contact with the lid on removal thereof from vessel and container.

The feeding of hose connections through the wall of the container in a liquid-tight manner can be achieved in a simple way by providing the wall with pipe connections projecting outwards and inwards, and on which both on the outside and on the inside hose connections can be placed, the hose connections in the container being connected to the operating head of the instrument to be treated.

The device according to the invention can now be further improved according to the invention in that the channel system comprises a detachably fitted vertical pipe with several crosswise branch pipe connections and with a downward-directed pipe connection, to which pipe connections hoses are detachably connected, part of said hoses running via a shutoff valve to a tank, and another part running to a pump for each hose, at least one of which pumps is connected to the bottom end of the vessel, and at least one of which is connected to the head of the instrument to be treated, while on the downward-directed pipe connection a hose with shutoff valve is connected. This vertical pipe, which is detachable and can thus be cleansed, now forms the central part to which one or more vessels with treatment media can be connected on one side by means of hoses with shutoff valves to be placed on the pipe connections, while the hoses serving for infeed of the media from a tank to the central pipe are provided with shutoff valves and the hoses serving for infeed of a medium or of several media from the central pipe to the outside and inside of the endoscope to be treated run via pumps. The central pipe has at its bottom end a discharge which is also provided with a valve, and can have at the top end a pipe connection with shutoff valve and connection to a source of gaseous medium under pressure, for example compressed air.

The various tanks can contain: a detergent, a disinfectant, alcohol, water or other media, while the tank provided under the discharge of the central pipe is for taking used media. One of the branch pipe connections can be connectable to the water mains for the supply of filtered water, in which case a separate tank with water is not required.

Provision can be made in the pipes running from the pumps to the container and then to the head of the endoscope to be treated for pressure sensors which provide information on the resistance met by the medium flowing through the internal channels of the endoscope. If hoses are used, these can be sensors which react to the expansion of the hose.

All shutoff valves are preferably in the form of electromagnetically operable hose clips, and all pumps are preferably reversible hose pumps. The hoses running from the pipe connections of the central pipe to the various connection points, which are the bottom end of the helical or spiral vessel, or the connections on the wall of the container, can then be continuous hoses which are easy to remove from the hose clips or from the hose pumps for cleaning, and whose interior cannot be soiled by the clips or pumps.

One has in this way obtained a device of which all essential parts which come into contact with the media, and thus also with the constituents carried away by it and thus removed from the endoscope, can themselves be completely sterilized and require little space for the purpose. The helical or spiral vessel is detachable and can easily be placed in an autoclave. The same applies to the detachable container. The hoses can also be cleaned or replaced, and this applies to the central pipe with pipe connections too.

It is pointed out that European Patent Application 0,084,342, which has been laid open for inspection, discloses a washing device for cleansing an elongated flexible medical instrument such as an endoscope, said known device also having a container in which the endoscope can be placed in the form of a spiral, but on a grid. This is a washing machine with which washing liquid can be sprayed from rotating sprayers against the top and bottom face of the endoscope. The overall height of this device is small, but it is not possible to disinfect the inside of the endoscope or the like with this device.

The device according to the invention makes it possible to operate pumps and hose clips according to a desired programme and thereby permits the carrying out of any desired cleansing programme, and through use of the central pipe this programme can have greater flexibility than that of the device mentioned at the beginning.

The invention will now be explained in greater detail with reference to the drawings.

Fig. 1 shows schematically a front view of the device according to the invention, with the front panel removed.

Fig. 2 shows a side view of Fig. 1.

Fig. 3 shows a modified embodiment of a part in cross section.

Fig. 4 is a top view of Fig. 3.

Fig. 5 shows another embodiment in side view.

Fig. 6 is a top view of Fig. 5.

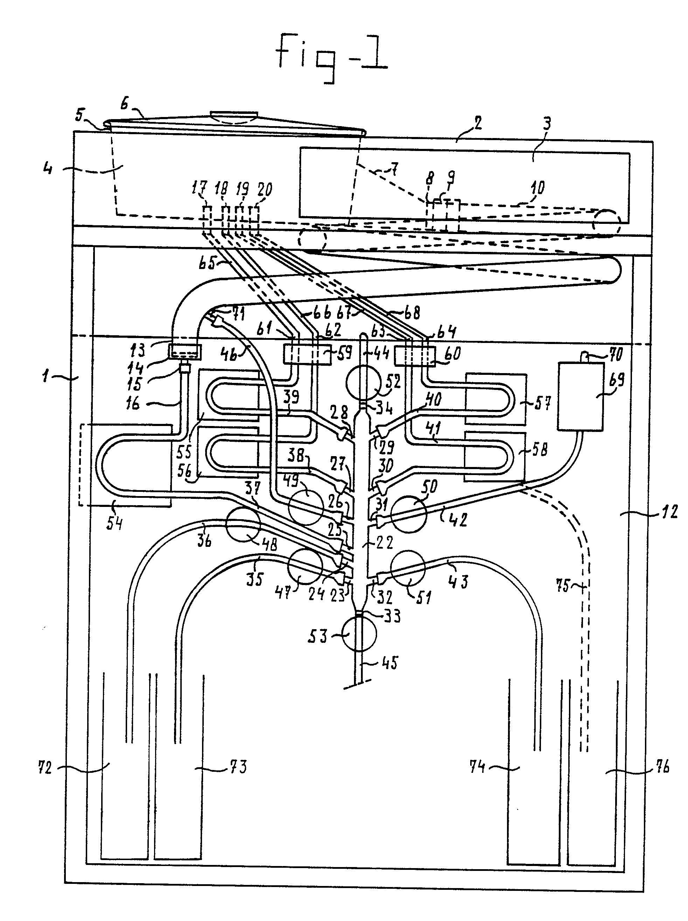

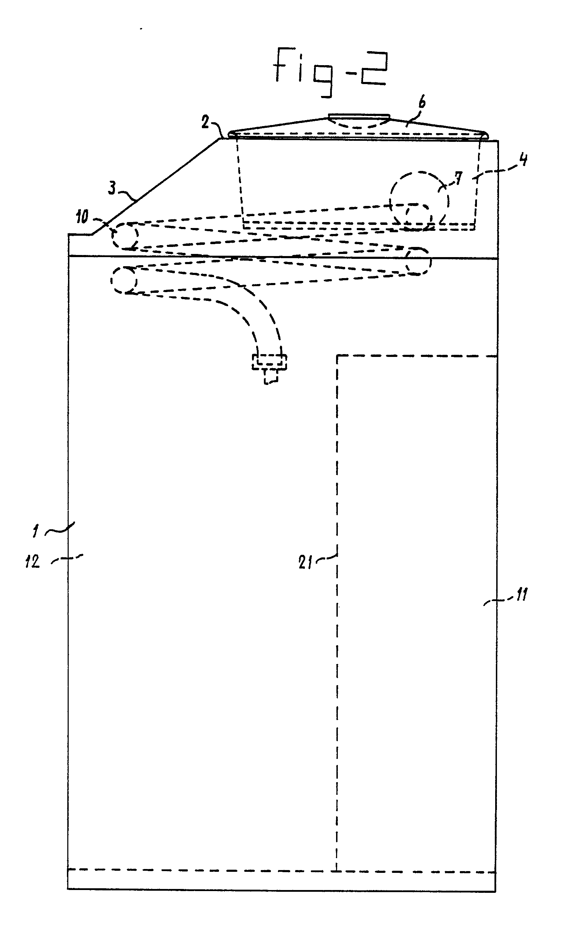

Figs. 1 and 2 show a cabinet 1 with a top leaf 2 which is at worktop height. This top leaf has a slanting part 3 for a control panel.

Located in the top leaf 2 is an opening in which is placed a pan-shaped container 4, which with its top edge 5 rests on the edge of the opening in the top leaf 2, and which is closed by a lid 6. This container 4 has a funnel-shaped connection piece 7 ending in a cylindrical end which fits in clamping fashion into a socket 9 on the top end of a tubular vessel 10 to be described in greater detail.

The cabinet 1 has a space 11 containing a preferably electronically controlled control system which can be programmed from the control panel 3 depending on the selection of washing programme and/or chosen disinfection programme which one wishes to carry out. The essence of the invention does not lie in the electronic control system or in the control panel, but in the parts of the device to be described in greater detail below.

The cabinet also has a space 12 which is accessible on removal of the front wall, as shown in Fig. 1, and in which the most important parts of the device according to the invention are accommodated.

The tubular vessel 10 runs essentially helically from the top end at the connecting socket 9 to the bottom end 13, which opens out into the cabinet space 12. Connected to this bottom end 13 by means of a screw coupling 14 with hose fitting 15 is a hose 16.

The container 4 is provided with four pipe connections 17, 18, 19 and 20, which project both inwards and outwards, and to which hose connections can be connected.

After removal of the lid 6, an endoscope to be treated can easily be inserted in the then open container, the flexible part being introduced through the funnel 7 into the helical pipe 10. The container has such a volume that the head of the endoscope can be fully accommodated therein and hose connections can be connected between the pipe connections 17 to 20 and the connections in the head of the endoscope.

Fixed detachably in the cabinet space 12 against an inside wall 21 is a vertical central pipe 22 which is provided with laterally projecting pipe connections 23 to 32, a vertically downward-directed pipe connection 33 and an upward-directed pipe connection 34.

Connected to all pipe connections are hoses, indicated by the numbers 35 to 46.

The hoses 35, 36, 42, 43, 44, 45 and 46 run through hose clips. These hose clips 47 to 53 are not shown in any further detail. They consist of electromagnetically operable clips which are known per se, and which either close or open the hose.

The pipe connection 33 at the bottom end of the central pipe 22 ensures discharge via hose 45 and hose clip 53.

The pipe connection 34 at the top end with hose 44 and hose clip 52 can be connected to a source (not shown) of gaseous medium under pressure, such as air.

Hose 37 runs through a hose pump 54 and is connected by part 16 to the bottom end 13 of the helical tubular vessel 10. This hose pump is capable of quickly feeding in or discharging liquid.

The hoses 38 and 39 run through hose pumps 55, 56, and the hoses 40 and 41 through hose pumps 57, 58 respectively.

The hoses coming out of the hose pumps 44 to 58 run via pressure sensors 59, 60 to connection points 61 to 64, from where hoses 65 to 68 can run to the pipe connections 17 to 20.

Hose 42 is connected to a filter 69, which is connected to the water mains, as shown at 70.

The hose 46 runs from the connection 26 via the hose clip 49 to a connecting stub 71 near the bottom end of the helical vessel 10.

When the hose clip 50 is open, water can come out of the mains 70 through filter 69 and hose 42 into the central pipe 22. This water is under mains pressure. It can go into the vessel through hose clip 49 and hose 46. With the pump 54 it is possible to feed to the vessel liquids which are not supplied under pressure via the helical vessel 10, and the liquid goes into the container 4 connected on the top of this vessel. An instrument placed therein can then be completely flushed with water. The container can have in it a level regulator (not shown) by means of which the action of the pump 54 can be stopped.

The infeed of washing liquid from the tank 72 can take place after the opening of the valve 48. Washing liquid can be sucked in via the hose 36 by means of, say, the pump 54 and then goes into the central pipe 22, can be mixed with water and by means of the pump 54 can be fed to the vessel 10. Complete flushing of the exterior of the endoscope is possible in this way, including the head in the container 4.

If air is supplied to the central pipe 22 through the pipe 44 and valve 52, this air can also pass via the pump 54 into the vessel or through the open valve 49 and the hose 46. The liquid contents of the tubular vessel 10 can now thereby be put into motion in such a way that at places where the endoscope may be lying against the wall of the vessel this contact is broken and good cleansing is therefore possible. Since the liquid can carry out an eddying movement, the cleansing is accelerated.

With the pumps 55, 56, 57 and 58, liquid can be fed from the central pipe 22 via the hoses 38, 39, 40 and 41 to the hoses 65-68, and thereby to the pipe connections 17 to 20, and via hoses connected thereto to the head of the endoscope to be treated, so that the internal channels thereof can be cleansed. Thereby the connection can by means of the hose 46 with open valve 49 ensure that circulation takes place. This can be done with the washing liquid from the tank 72, but it can also be done with a disinfectant, for example from the tank 73 or with alcohol from tank 74. The dashed lines 75 show that instead of a mains connection 70, it is also possible to fetch water from a separate tank 76.

The pressure sensors 59, 60 can be used to find out whether resistance is encountered in the channels of the endoscope to be flushed.

The system can be emptied completely by reversal of the working direction of the hose pumps, in particular of the main pump 54. The hose pumps are known per se. They have a rotor with rollers which can press the hose shut, and which on displacement over the hose bring about the movement. At a standstill they function as shutoff valves. With such hose pumps the hose can easily be inserted or removed laterally by hand. The latter applies also to the hose clips known per se.

All parts, such as the helical container 10, the container 5, the central pipe 22 and all hoses connected thereto are quick to remove, replace and clean. The pumps and hose clips need not be cleaned, since they do not come into contact with the interior of the hoses.

In the embodiment shown in Figs. 1 and 2 the container 4 is a container which can be closed liquid-tight with a lid.

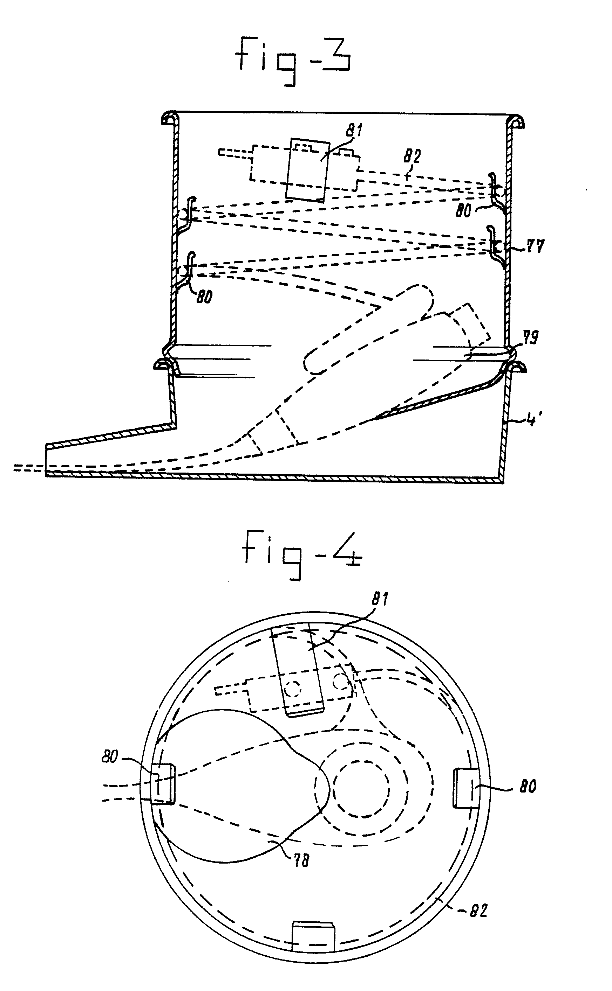

Figs. 3 and 4 show now that this container 4' can be provided with an extension piece 77 in the form of a bowl having in the bottom an opening 78 for inserting therethrough an endoscope 79. Said bowl 77 can be placed with sealing effect on the top rim of the container 4', can be open at the top, but can also be closed there with the lid 6. This bowl has in the wall supports 80 and 81 for supporting the optical fibre connected on the head of the endoscope. This embodiment permits partial flushing of the head of the endoscope and of the part projecting into the helical vessel, and also full cleansing of the internal channels. The optical fibre resting in the supports is indicated by 82. This is the part which has to be connected to the light source.

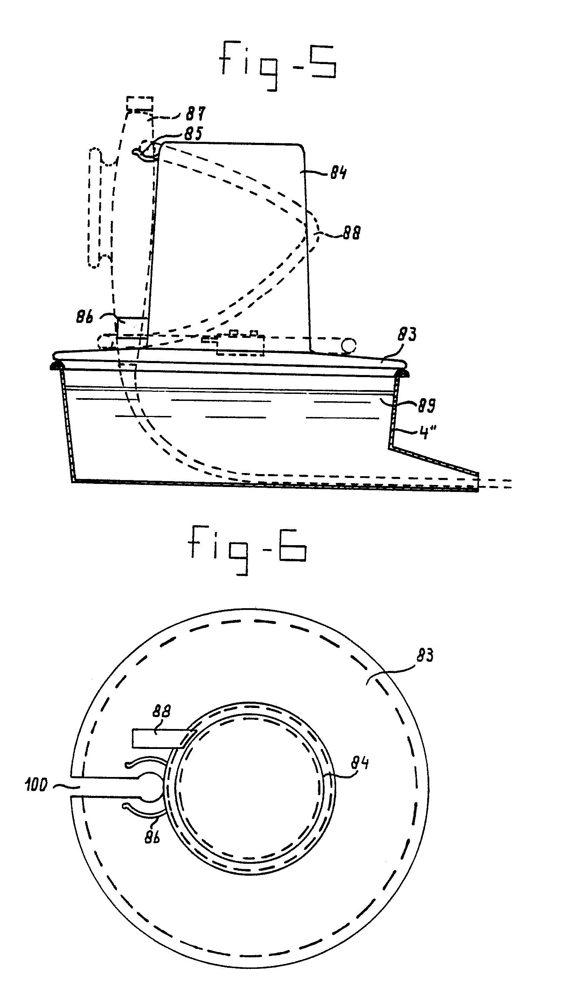

Figs. 5 and 6 show an embodiment in which

the container 4″ is shut with a lid 83 which is provided with an upward-facing bell-shaped part 84 with support elements 85, 86 for the head 87 of an endoscope. The bell-shaped part here serves to support the optical fibre 88, which also rests on the top side of the lid 83.

In this embodiment the hoses for the infeed of media to the internal channels of the endoscope can be connected outside the container 4′ on the head of the endoscope. Cleansing of the outside of the endoscope is possible only insofar as the liquid level 89 in the container 4″ is adequate. The part supported on the lid and located on the outside of the container 4″ cannot be cleansed with this embodiment.

In this embodiment this is no problem, because after cleaning and sterilization of the flexible part the endoscope can be removed from the vessel by lifting off the lid 83 and unhooking the head 87 from the supports 85, 86 without the cleansed and sterilized flexible part coming into contact with the non-sterilized top edge of the container 4″. This arises from the large dimensions of the opening of the container 4″ and from the fact that this opening is at worktop level.

The lid 83 in this embodiment also has a slit 100 for lateral insertion or removal of the flexible part of the endoscope to be treated.

**Claims**

1. Device for cleansing and disinfecting an elongated medical instrument of the type with a long flexible part, such as an endoscope, said device comprising an elongated tubular vessel having a cross section which is greater than that of the instrument to be treated, and having a length suitable for taking the flexible part of the instrument to be treated, said vessel having a top end and a bottom end, of which the top end is suitable for introducing an instrument to be treated, and of which the bottom end can be connected to a treatment liquid discharge, said device also being provided with one or more tanks with treatment media and with a channel system with valves, pumps and hoses for the infeed of said media to the inner channels of the instrument to be treated and/or to the top end of the tubular vessel, characterized in that the vessel is detachably connected at the top end to a container whose shape and/or volume is suitable for the accommodation of the operating head of the instrument to be treated.

2. Device according to Claim 1, characterized in that the vessel between top and bottom end follows a path provided with curves, whose width in the horizontal direction is greater than the vertical distance between top and bottom end.

3. Device according to Claim 2, characterized in that the path is helical.

4. Device according to Claim 2, characterized in that the path is spiral.

5. Device according to one or more of the preceding Claims 1-4, characterized in that the container is closable so that it is liquid-tight, and provision is made in the wall for sealed passages for the supply of treatment media through the wall to the container, in particular to an operating head of an instrument for cleansing located in the closable container.

6. Device according to one or more of the preceding Claims 1-5, characterized in that the container is provided closably with an extension piece which can be placed on the top rim of the container and forms a support for the head of the instrument to be treated, and which has a passage for the part of the instrument following the head.

7. Device according to Claim 6, characterized in that the extension piece is a bowl with an opening in the bottom carrying the head to allow through the instrument, and with supports for the optical fibre of the instrument.

8. Device according to Claim 6, characterized in that the extension piece is a lid with an upward-directed support for supporting the head of the instrument to be treated on the outside of the lid, and also having a support for the optical fibre of this instrument, and having a feed-through slit for the part of the instrument connecting to the head of the instrument.

9. Device according to one or more of the preceding claims, characterized in that the channel system comprises a detachably fitted vertical pipe with several crosswise branch pipe connections and with a downward-directed pipe connection, on which pipe connections hoses are detachably connected, part of said hoses running via a shutoff valve to a tank, and another part running to a pump for each hose, at least one of which pumps is connected to the bottom end of the vessel, and at least one of which is connected to the head of the instrument to be treated, while on the downward-directed pipe connection a hose with shutoff valve is connected.

10. Device according to Claim 9, characterized in that the vertical pipe is provided at its top end with a pipe connection for connecting thereto a hose with shutoff valve, said hose being connected to a source of gaseous medium under pressure.

11. Device according to Claim 9 or 10, characterized in that one branch pipe connection is connectable to the water mains.

12. Device according to Claim 9, 10 or 11, characterized in that the pipes or hoses running upstream of the pumps to the container are provided with pressure sensors.

13. Device according to one or more of the preceding Claims 9 to 12, characterized in that all shuroff valves are electromagnetically operable hose clips.

14. Device according to one or more of the preceding Claims 9-13, characterized in that all pumps are reversible hose pumps.

Fig-1

0 271 157

Fig-2

# Fig-3

81
82
80
77
80
79
4'

# Fig-4

81
80
80
78
82

Fig-5

Fig-6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | FR-A-2 323 399 (RIWOPLAN) * Page 1, lines 21-40; page 2, lines 8-26; page 3, line 29 - page 4, line 34; figures 1-4 * | 1,2,4-6 | A 61 B 1/12 A 61 L 2/18 |
| X | FR-A-2 462 897 (MUTOH CORP., LTD) * Page 3, line 15 - page 7, line 36; figures 1,2 * | 1,2,4,5 ,13 | |
| X | US-A-3 963 438 (A.V. BANEZ) * Abstract; column 1, line 55 - column 2, line 66; figures 1,2 * | 1,5,6 | |
| A | FR-A-2 332 074 (RIWOPLAN) * Page 2, lines 6-15,21-35; page 3, line 1 - page 4, line 25; figure 1 * | 1,9-11, 13 | |
| A | DE-A-3 416 743 (W. GRIESAT) * Abstract; page 1, lines 3-6; claims 4,18,22,28,34,37; page 9, lines 6-12; page 11, lines 27-34; page 13, lines 26-35; page 15, lines 1-7; page 16, line 5 - page 17, line 5; page 18, lines 21-26; page 19, lines 6-16; figure 1 * | 1,8,12, 14 | |
| A | DE-A-3 443 912 (MIELE & CIE) * Abstract; pages 1,2, claims 1,3,4; figures 1-3 * | 1-5,10, 11 | TECHNICAL FIELDS SEARCHED (Int. Cl.4) A 61 B A 61 C A 61 L B 08 B |

The present search report has been drawn up for all claims

| Place of search | Date of completion of the search | Examiner |
|---|---|---|
| THE HAGUE | 15-02-1988 | RIEB K.D. |